# EUROPÄISCHE PATENTSCHRIFT

(11) **EP 2 023 814 B1**
(45) Veröffentlichungstag und Bekanntmachung des Hinweises auf die Patenterteilung: **06.01.2016**
(21) Anmeldenummer: 07764330.2
(22) Anmeldetag: 16.05.2007
(51) Int. Cl.: A61B 5/06

(54) **ANORDNUNG ZUR BERÜHRUNGSLOSEN DEFINIERTEN BEWEGUNG MINDESTENS EINES MAGNETISCHEN KÖRPERS**
ARRANGEMENT FOR NON-CONTACT DEFINED MOVEMENT OF AT LEAST ONE MAGNETIC BODY
DISPOSITIF PERMETTANT UN MOUVEMENT DÉFINI, SANS CONTACT, D'AU MOINS UN CORPS MAGNÉTIQUE

(30) Priorität: 16.05.2006 DE 102006023428; 20.06.2006 DE 102006028704
(43) Veröffentlichungstag der Anmeldung: 18.02.2009
(73) Patentinhaber: Fraunhofer-Gesellschaft zur Förderung der angewandten Forschung e.V., 80686 München (DE)
(72) Erfinder: ANDRÄ, Wilfried, 07749 Jena (DE); BRAND, Michael, 09518 Grossrückerswalde (DE); LAUSCH, Holger, 07743 Jena (DE); WERNER, Christoph, 07743 Jena (DE)
(74) Vertreter: Oehmke, Volker
(86) Internationale Anmeldenummer: PCT/DE2007/000910
(87) Internationale Veröffentlichungsnummer: WO 2007/131503

(56) Entgegenhaltungen:
- WO-A-2004/075749
- US-A1- 2004 138 552
- US-A1- 2005 183 733
- US-B1- 6 168 780

## Beschreibung

Die Erfindung betrifft eine Anordnung zur berührungslosen definierten Bewegung mindestens eines magnetischen Körpers gemäß der Gattung der Patentansprüche. Sie ist in allen Bereichen der Technik und Medizin anwendbar, in denen es darum geht, den Weg, die Position und die Orientierung von magnetischen Körpern durch unzugängliche Kanäle und an unzugänglichen Stellen zu ermitteln.

Gattungsgemäße Lösungen sind beispielsweise aus den Schriften US 2004/0138552 A1 und US 6,168,780 B1 bekannt. Die US 2004/0138552 A1 betrifft eine Anordnung zur Beeinflussung eines Vehikels in einer Umgebung wie dem Körperinneren einer Person, wobei an dem Vehikel ein Magnet angebracht bzw. in diesem angeordnet ist. Mittels eines externen Magnetfelds (Primärmagnetfeld) kann das Vehikel gesteuert bewegt werden. Neben dem Vehikel und einer Magnetfeldquelle sind ein Magnetfeldsensor und eine Auswerte- und Steuereinheit zur Auswertung erfasster Signale des Magnetfeldsensors und zur Steuerung des Vehikels durch Veränderungen des Magnetfelds vorhanden.

Durch die US 6,168,780 B1 ist ebenfalls ein Vehikel offenbart, das insbesondere den Gastrointestinaltrakt eines Lebewesens passieren soll und mittels dem Durchgangs- und Verweilzeiten des Vehikels ermittelbar sind. In einem sphärischen Bauelement (Kapsel) ist ein magnetisiertes Material eingeschlossen. Das Bauelement ist von einem Gehäuse umschlossen. Zwischen dem Bauelement und dem Gehäuse ist eine Flüssigkeit vorhanden, sodass das Bauelement in der Flüssigkeit gelagert ist. Eine Steuerung und Ortung kann durch ein externes Magnetfeld (Primärmagnetfeld) erfolgen. Durch den magnetischen Körper kann ein Sekundärmagnetfeld bewirkt sein.

Der Erfindung liegt die Aufgabe zugrunde, eine Anordnung zur berührungslosen definierten Bewegung eines Messobjektes zu schaffen, die universell einsetzbar ist zum Positionieren und Orientieren des Messobjektes (des magnetischen Körpers) wie auch zur Feststellung seiner Lage im Raum. Ferner soll die Anordnung zur Energiegewinnung und Energieübertragung und zur Feststellung gewisser physikalischer und/oder chemischer Eigenschaften des Messobjektes und seiner unmittelbaren Umgebung geeignet sein. Schließlich soll die Anordnung für spezielle Anwendungen sehr kleine und gedrängte Bauweisen ermöglichen.

Gemäß der Erfindung wird diese Aufgabe durch die kennzeichnenden Merkmale des Anspruchs 1 gelöst und durch die Unteransprüche vorteilhaft ergänzt.

Die Aufgabe wird daher in einer Anordnung zur berührungslosen Bestimmung der Orientierung und Positionierung mindestens eines Gehäuses gelöst, in dem ein magnetischer Körper in einer Kapsel in wenigstens einer Dimension frei beweglich gelagert ist und / oder zur berührungslosen Energieübertragung in das Gehäuse, dessen Bewegung damit von der Bewegung des mit der Kapsel starr verbundenen Gehäuses unabhängig und getrennt ist. Eine erfindungsgemäße Anordnung ist ferner dadurch gekennzeichnet, dass der ein magnetisches Moment aufweisende magnetische Körper im Primärmagnetfeld mindestens eines definiert beweglichen Permanentmagneten angeordnet ist, der magnetische Körper ein vom magnetischen Körper ausgehendes Sekundärmagnetfeld aufweist, wobei bei einer Bewegung des Primärmagnetfelds relativ zum magnetischen Körper Veränderungen der Ausrichtung des Sekundärmagnetfelds bewirkt sind, und dadurch der magnetische Körper definiert bewegt ist und dass die Kapsel mit einer dreidimensional wirkenden Spulenanordnung versehen ist, die an der Kapsel befestigt ist.

Mit anderen Worten: Das vom Permanentmagneten ausgehende erste magnetische Streufeld (Primärmagnetfeld) bewirkt bei seiner Bewegung relativ zum magnetischen Körper (Messobjekt) Veränderungen der Ausrichtung des zweiten magnetischen Streufeldes (Sekundärmagnetfeld), die mit Hilfe eines Magnetfeldsensors hinsichtlich der Stärke, der Richtung und des Phasenwinkels zum ersten Streufeld in einer Ebene oder im Raum gemessen werden. Da das Messobjekt oder die Messobjekte in Kapseln frei beweglich gelagert sind, können sie mit Hilfe des sich bewegenden Permanentmagneten (Feldgeber) gezielt ausgerichtet oder anderweitig bewegt werden. Der Permanentmagnet selbst kann rotationssymmetrisch gestaltet sein und ist radial magnetisiert, d. h. bspw. an den Enden eines Zylinderdurchmessers befinden sich unterschiedliche Polungen. Die Bewegung des Permanentmagnets kann durch einen Motor geschehen, der den Rotationskörper um seine Symmetrieachse dreht. Das Messobjekt ist vorteilhaft als stabförmiger oder besser als kugelförmiger Dipol gestaltet, der sich in einer Lagerflüssigkeit in der Kapsel dreidimensional frei bewegen kann. Aus dem Phasenwinkel zwischen dem Primär- und dem Sekundärmagnetfeld ergibt sich ein Nachlauf des Sekundärmagnetfeldes, von dem auf die Viskosität der Lagerflüssigkeit geschlossen werden kann. Es versteht sich von selbst, dass die freie Beweglichkeit des Dipols auch durch eine kardanische Lagerung gewährleistbar ist.

Nach den Freiheitsgraden der Bewegung des magnetischen Körpers in der Kapsel richtet sich die Anzahl der Magnetfeldsensoren bzw. die Gestaltung des Magnetfeldsensors, der vorteilhaft ein Magnetometer ist. Der Magnetfeldsensor/die Magnetfeldsensoren können beliebig im Raum angeordnet oder gelagert sein; sie können auch mit der Zeit ihre Positionen ändern und mit dem Permanentmagneten fest verbunden sein bzw. sich synchron mit diesem bewegen. Vorteilhaft sind sie jedoch fest im Raum angeordnet.

Die Messung des Sekundärmagnetfeldes kann in der Weise erfolgen, dass die Veränderung gegen das Primärfeld am Sensorort (Referenzfeld) bestimmt wird. Beim Drehen des Permanentmagnets und damit des Primärmagnetfeldes gegenüber dem Magnetfeldsensor ändert sich das Referenzfeld in Abhängigkeit vom Winkel zur Sensorachse. Vorteilhaft ist es jedoch, dass Primärmagnetfeld am Sensorort derart zu kompensieren, dass sein Betrag konstant oder vorzugsweise Null ist. Dies kann günstigerweise mit Hilfe einer permanentmagnetischen Kompensationseinrichtung geschehen, welche aus einer Permanentmagnetanordnung besteht, die mit dem rotationssymmetrischen Permanentmagneten fest verbunden und um dieselbe Achse drehbar gelagert ist und die am Ort des Magnetfeldsensors ein dem Primärmagnetfeld entgegengesetzt gerichtetes Feld gleicher Stärke erzeugt. Die Kompensation des Primärmagnetfeldes kann auch mit einer elektromagnetischen Kompensationseinrichtung vorgenommen werden, bei der mindestens eine elektrische Spule koaxial zur Drehachse des Permanentmagnets angeordnet und mit diesem synchron drehbar ist. Auch in diesem Fall wird am Sensorort ein dem Primärmagnetfeld vorzugsweise entgegengesetztes Feld gleichen Betrags erzeugt.

Es können auch mehrere erfindungsgemäße Anordnungen nacheinander so angeordnet sein, dass das jeweilige Sekundärmagnetfeld als Primärmagnetfeld zur Anregung weiterer magnetischer Körper (Messobjekte) dient.

Die erfindungsgemäße Anordnung kann nicht nur zur Übertragung von Bewegungen zur Ortung und Anzeige von Positionen, sondern auch zur Orientierung bzw. Anzeige der Orientierung des magnetischen Körpers bzw. eines ihm zugeordneten Gehäuses dienen, da die den freibeweglichen magnetischen Körper beinhaltende Kapsel von einer vorzugsweise dreidimensional wirkenden Spulenanordnung umgeben ist, die an der Kapsel befestigt ist. Bei Bewegung des magnetischen Körpers in der Kapsel wird nicht nur die Lage des Sekundärfeldes gegenüber dem Primärfeld verändert, sondern es werden auch in der Spulenanordnung Ströme induziert, die einer Auswerte- und Steuereinheit zugeführt werden, welche daraus die Orientierung des magnetischen Körpers im Raum ermittelt und ggf. anzeigt. Umgekehrt kann die Spulenanordnung in geeigneter Weise bestromt werden, um dem magnetischen Körper eine gewünschte räumliche Orientierung zu geben.

Spulenanordnung und magnetische Körper können auch einen Generator zur Energiegewinnung bilden, wobei die Generatorenergie einem Speicher oder mindestens einem Energieverbraucher zugeordnet wird. Dabei sind die einzelnen Spulen der Spulenanordnung vorteilhaft in Reihe geschaltet, um einen maximalen Generatorwirkungsgrad zu erzielen.

Zur Bewegungsübertragung ist die Generatorkapsel (Kapsel mit dem magnetischen Körper) vorteilhaft in einem Gehäuse einerseits fest angeordnet, in dem andererseits eine Übertragungskapsel befestigt ist. Die Übertragungskapsel weist eine Drehachse auf, welche mit der Drehachse des magnetischen Körpers in der Generatorkapsel zumindest angenähert fluchtet.

Die Auswerte- und Steuereinheit kann auch mit der Spulenanordnung derartig elektrisch verbunden sein, dass von ihr aus die Spulenanordnung bestromt wird, so dass in der Kapsel ein Magnetfeld erzeugt wird, das sich dem Sekundärmagnetfeld des magnetischen Körpers zur definierten Bewegung dieses Körpers und der Kapsel überlagert.

Weitere vorteilhafte Gestaltungen und Wirkungsweisen der Erfindung sind den nachfolgenden verbalen und zeichnerischen Darstellungen entnehmbar.

Die Erfindung wird nachstehend an Hand der schematischen Zeichnung von vier Ausführungsbeispielen näher erläutert. Es zeigen:
- Figur 1: den Aufriss eines ersten Ausführungsbeispiels der Erfindung,
- Figur 2: einen Grundriss des ersten Ausführungsbeispiels der Erfindung,
- Figur 3: eine abgewandelte Kapsel als zweites Ausführungsbeispiel der Erfindung,
- Figur 4: einen Längsschnitt eines dritten Ausführungsbeispiels der Erfindung und
- Figur 5: einen Längsschnitt eines vierten Ausführungsbeispiels der Erfindung, bei dem die Erfindung sowohl der Positionierung und Orientierung als auch der Energiegenerierung und Übertragung dient.

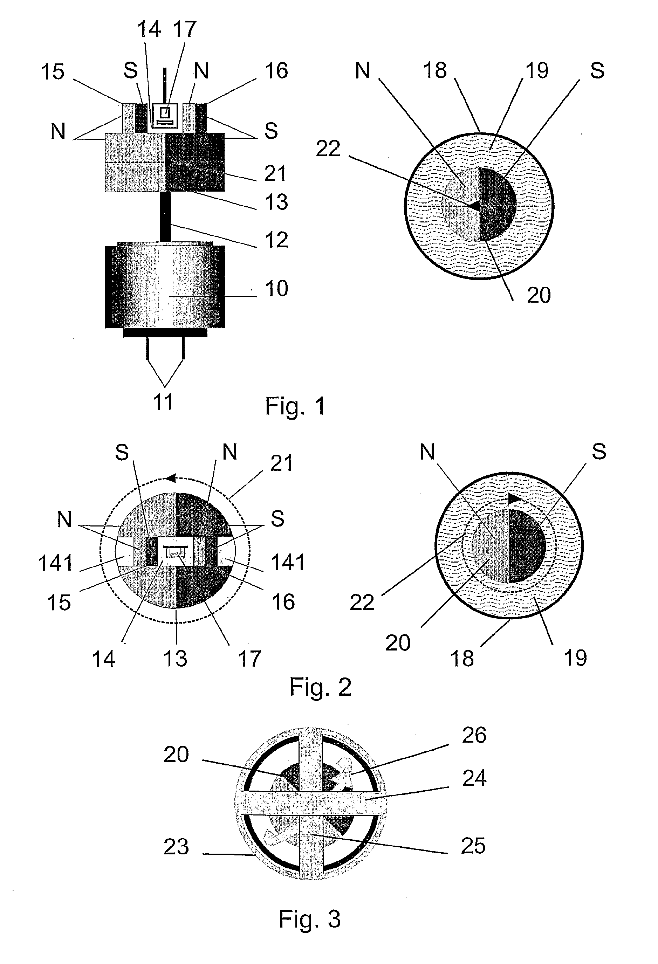

In den Figuren 1 und 2 wird von einem Motor 10 mit elektrischen Anschlüssen 11 über eine Welle 12 ein rotationssymmetrischer Permanentmagnet (zylindrischer Feldgeber) 13 in Drehungen um eine geometrische Achse X-X (verdeckt) versetzt. Der Permanentmagnet 13 ist radial magnetisiert und besitzt eine Nordpolhälfte N und eine Südpolhälfte S. Am Permanentmagnet 13 sind einem Zwischenraum 14 einschließende quaderförmige kleinere Permanentmagnete 15, 16 über eine Halterung 141 befestigt, deren Pole ebenfalls N und S sind. Im Zwischenraum 14 ist ein im vorliegenden Fall dreidimensional wirkender Magnetfeldsensor 17 von den Magneten 13, 15, 16 getrennt und starr angeordnet. Der Permanentmagnet 13 erzeugt ein Primärmagnetfeld, in dem sich eine unmagnetische Kapsel 18 befindet, in welcher ein in einer nichtmagnetischen Flüssigkeit 19 frei schwimmender magnetischer Körper (kugelförmiger Dipol) mit den Polen N und S gelagert ist, dessen magnetisches Streufeld (Sekundärmagnetfeld) mit Hilfe des Magnetfeldsensors 17 gemessen werden soll. Beim Drehen des Permanentmagnets 13 in Richtung eines Pfeiles 21 dreht sich der magnetische Körper 20 in Richtung eines Pfeiles 22, also entgegengesetzt. Da die Permanentmagnete 15, 16 ein Magnetfeld aufbauen, das im Zwischenraum 14 dem Primärmagnetfeld im Betrag gleich, aber entgegengesetzt ist, wirkt auf den Magnetfeldsensor 17 nur das vom magnetischen Körper 20 erzeugte, sich am Primärmagnetfeld ausrichtende Sekundärmagnetfeld.

Das Ausführungsbeispiel der Figuren 1 und 2 dient sowohl der Bewegungsübertragung auf den magnetischen Körper 20 als auch der Ortung des magnetischen Körpers 20 mit Hilfe des Magnetfeldsensors 17. Die Erfindung ist nicht an die dargestellten Gestaltungen und Anordnungen des Permanentmagneten 13, der Kompensationseinrichtung 15, 16, des Magnetfeldsensors 17 und des magnetischen Körpers 20 gebunden.

Figur 3 zeigt abweichend von den Figuren 1 und 2 eine Kapsel 18, der eine dreidimensionale wirkende Spulenanordnung mit Induktionsspulen 23, 24, 25 zugeordnet ist. In der Kapsel 18 ist ein kugelförmiger Dipol 20 in einer Flüssigkeit 19 frei beweglich gelagert, was durch einen Pfeil 26 angedeutet ist. Wird der Dipol 20 ähnlich wie in den Figuren 1 und 2 bewegt, so induziert er in den Spulen 23, 24, 25 im Allgemeinen unterschiedliche Ströme, die einer nicht dargestellten Auswerteeinheit zugeführt werden, welche aus den Strömen die Orientierung des Dipols 20 bzw. der Kapsel 18 im Raum ermittelt und anzeigt.

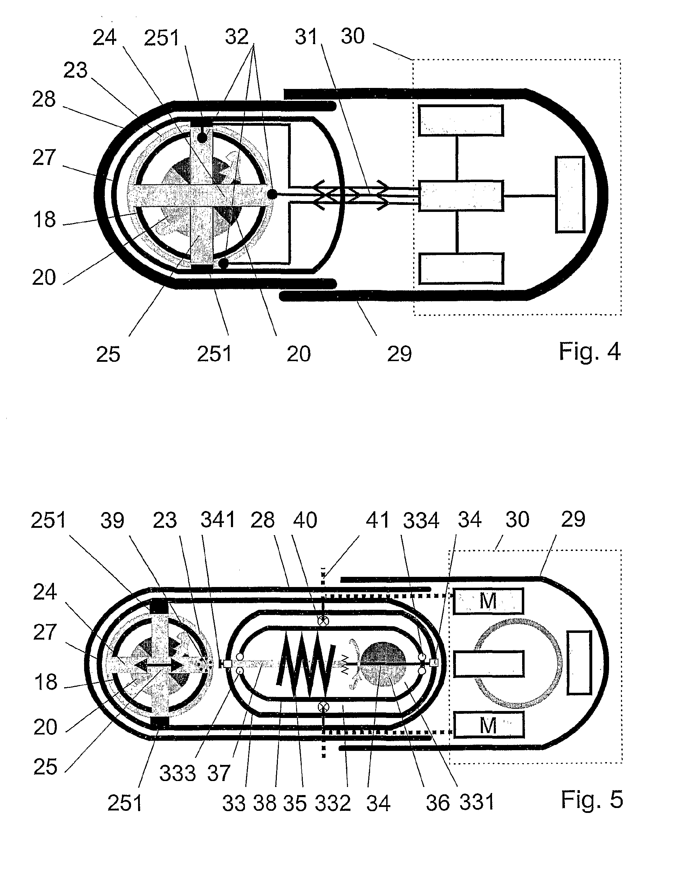

In Figur 4 ist wieder ein magnetischer Körper 20 in einer Kapsel 18 frei drehbar gelagert, die von einer Spulenanordnung 23, 24, 25 umgeben ist, deren Befestigungen an einem Gehäuse mit 251 bezeichnet sind. Kapsel 18 und Spulenanordnung 23, 24, 25 sind in einem Gehäuse 27 befestigt, das seinerseits von einer äußeren Gehäuseschale 28 umgeben ist. In einer zweiten äußeren Gehäuseschale 29 ist eine Auswerte- und Steuereinheit 30 angeordnet, zu der elektrische Verbindungen 31 mit Kontakten 32 von der Spulenanordnung 23, 24, 25 bestehen. Beide Gehäuseschalen 28, 29 sind teleskopartig ineinander schiebbar. Durch die elektrischen Verbindungen 31 wird die in der Spulenanordnung 23, 24, 25 durch Bewegung des magnetischen Körpers 20 gewonnene elektrische Energie zum Antrieb von Elektromotoren mit angeflanschten Bewegungstraktoren (nicht dargestellt) und damit zur ferngesteuerten Bewegung benutzt. Ebenso kann die gewonnene elektromagnetische Energie zur Energieversorgung von Kapselkomponenten, wie bspw. Sensoren, Aktoren, Datenübertragungs- und Steuerungssystemen, Akkumulatoren usw. benutzt werden. Schließlich ist es möglich, die Kapsel 18 mit der sie umgebenden Spulenanordnung 23, 24, 25 ausschließlich für die Ortung und Lagebestimmung im Raum zu verwenden. Die elektrischen Verbindungen 31 sind zu dem Zweck vorteilhaft derart gestaltet, dass sie zu verarbeitende Signale von den Spulen 23, 24, 25 zur Auswerte- und Steuereinheit 30 transportieren und Steuersignale von der Auswerte- und Steuereinheit 30 über Kontakte 32 an die Spulen 23, 24, 25 zur Ausrichtung des Dipols 20 geben. Die Auswerte- und Steuereinheit 30 kann auch als ferngesteuertes System ausgestaltet sein.

In Figur 5 sind zwei äußere Gehäuseschalen 28, 29 teleskopartig ineinander geschoben. In der einen Gehäuseschale 28 befinden sich ein Schutzgehäuse 27 für die mit der Spulenanordnung 23, 24, 25 versehene Kapsel 18, in welcher der Dipol 20 frei drehbar gelagert ist, und eine längliche Übertragungskapsel 33. In Längsrichtung der Übertragungskapsel 33 ist in dieser mittig eine Welle 34 gelagert, auf der eine Förderschraube 35 und ein magnetischer Dipol 36 befestigt sind. Dieser auf der Welle 34 abseitig von der Kapsel 18 gelagerte Dipol 36 hat vorzugsweise ein vergleichsweise geringeres magnetisches Moment als der Dipol 20 in der Kapsel 18. Dem Dipol 36 ist auf der Übertragungskapsel 33 eine Induktionsspule 37 zugeordnet. Durch eine zur Welle 34 rotationssymmetrische Wandung 38 ist der Innenraum der mit Flüssigkeit gefüllten Übertragungskapsel 33 radial in einen inneren Teilraum 331 und einen äußeren Teilraum 332 geteilt, die über Steuerventile 333, 334 in Verbindung stehen. An ihrem der Kapsel 18 zugewandten Ende ist die Welle 34 mit einer Druckfläche 341 versehen, der an der Kapsel 18 ein Blähsack oder Schalter 39 gegenüber liegen. Abweichend von der Darstellung in Figur 5 kann die Förderschraube 35 selbst als magnetischer Dipol ausgebildet sein.

In der äußeren Gehäuseschale 29 befindet sich eine Auswerte- und Steuereinheit 30 die Sensoren, Motoren, Akkumulatoren, Datenverarbeitungs- und Übertragungsmittel und ggf. ein Flüssigkeitsreservoir umfasst.

Antriebsabgriffe 40 und Antriebsachsen 41 dienen der Übertragung der Energie, wenn die Spulenanordnungen 23, 24, 25 und der magnetische Körper 20 in der Kapsel 18 einen Generator zur Energiegewinnung bilden.

Wird außerhalb der Gehäuseschalen 28, 29 ein magnetisches Wechsel-, Dreh- oder Impulsfeld erzeugt, so richtet sich der magnetische Körper 20 in der Kapsel 18 nach dem jeweiligen Feld aus und rotiert genau um eine Achse, die rechtwinklig zum Primärfeld am Ort des Körpers 20 gerichtet ist, wodurch sich ein optimaler Ortungs- und Generatorwirkungsgrad ergibt. Der starr auf der Welle 34 der Übertragungskapsel 33 befestigte magnetische Dipol 36 rotiert dann optimal, wenn sich das außen angelegte magnetische Feld genau auf dessen Rotationsachse ausrichtet. In diesem Falle wird die Rotationsbewegung optimal über die Förderschraube 35 vorwärts oder rückwärts umgewandelt. Damit können auf beiden Seiten der Übertragungskapsel 33 unterschiedliche Flüssigkeitsdrücke generiert werden. Bei Verbindung der beiden Enden der Übertragungskapsel 33 mit einem Schlauchsystem arbeitet die Übertragungskapsel 33 als Pumpe. Je nach Pumprichtung können bspw. die Flüssigkeitsströme (nicht dargestellt) in dem Schlauchsystem für richtungsändernde Antriebssysteme verwendet werden, indem die Längsbewegung der Flüssigkeit in eine Drehbewegung umgewandelt wird. So können sich zwei oder mehrere Antriebsräder o. ä. in der Übertragungskapsel 33 in unterschiedliche Richtungen drehen, womit die Gesamtanordnung gezielt bewegt bzw. ferngesteuert werden kann.

Während sich die Rotationsachse des magnetischen Körpers 20 in der Kapsel 18 dem angelegten Magnetfeld frei anpasst und damit eine maximal genaue Ortung und optimale Generatorleistung erreicht, trifft dies für den magnetischen Dipol 36 auf der raumfesten Welle 34 nur dann zu, wenn die Drehachse des äußeren magnetischen Feldes mit der Rotationsachse des Dipols übereinstimmt. In diesem Fall ist das magnetisch zu ortende Drehfeld am größten. Neben der Ortung der Kapsel 18 im Raum kann damit auch eine Achsausrichtung der Kapsel 18 bestimmt werden, indem das äußere Magnetfeld dreidimensional so ausgerichtet wird, dass die Rotationsachsen von magnetischem Körper 18 und Dipol 36 sich in einer Flucht befinden und gegenseitig verlängern und auf diese Weise ein maximales Drehfeld zur Ortung generieren.

Die Anordnung der Kapsel 20 sowie der Übertragungskapsel 33 innerhalb des Gehäuses 32 kann so gestaltet werden, dass bei entsprechend hohen Drehzahlen des magnetischen Körpers 20 und der damit verbundenen Erwärmung der Lagerflüssigkeit 19 sich der Blähsack 39 ausdehnt und auf die Druckfläche 341 an der Welle 34 einen Druck ausübt, wodurch die Welle 34 fixiert und damit ihre Drehung verhindert wird. Dieser Effekt kann zur hochgenauen Ortung benutzt werden, weil nur ein Drehfeld im Gehäuse 32 erzeugt wird. Danach hebt sich bei geringerer Rotation des magnetischen Körpers 20 in der Kapsel 18 und damit ab Kühlung der Lagerflüssigkeit 19 der Blähsack 39 wieder von der Druckfläche 341 ab, die Welle 34 mit der Förderschraube 35 beginnt sich wieder zu drehen bis beide Rotationsachsen, wie oben dargestellt, übereinstimmen und die Achsausrichtung des Gesamtgehäuses bestimmt werden kann.

Ist auf der Welle 34 keine Förderschraube vorgesehen, dann wird die Anordnung zur Bestimmung der Ausrichtung der Gehäuseschalen 28, 29 mit ihrem gesamten Inhalt benutzt. In diesem Fall kann auch die Übertragungskapsel 33 sowohl mit als auch ohne zusätzliche Induktionsspule 37 ausgeführt werden. Die in einer oder beiden Kapseln 18, 33 gewonnene elektrische Energie kann auch über die Antriebsachsen 41 zum Antrieb von nicht dargestellten Elektromotoren mit angeflanschten Bewegungstraktoren und damit zur ferngesteuerten Bewegung genutzt werden.

Die erfindungsgemäße Anordnung ist geeignet, folgende Funktionen bzw. Anwendungen einzeln, getrennt, zeitlich versetzt und/oder parallel für die Messung von Parametern zur Bestimmung sowie zur Steuerung und Beeinflussung von Positionen und Zuständen des Messobjekts, Lagervolumens und Lagermediums zu realisieren:
- Ortung und Anzeige der Position des Messobjektes,
- räumliche Ausrichtung bzw. Angabe der Orientierung des Messobjektes,
- berührungslose Übertragung von Energie zum bzw. vom Messobjekt,
- Messung bestimmter physikalischer und chemischer Eigenschaften bzw. Zustände des Messobjekts, des Lagervolumens und des Lagermediums des Messobjekts,
- ferngesteuerte Beeinflussung bestimmter physikalischer und chemischer Eigenschaften bzw. Zustände des Messobjekts, des Lagervolumens und des Lagermediums, um bspw. Bewegungsabläufe, Öffnungsmechanismen und/oder Freisetzungsmechanismen auslösen zu können.

Alle in der Beschreibung und den nachfolgenden Ansprüchen dargestellten Merkmale können sowohl einzeln als auch in beliebiger Kombination miteinander erfindungswesentlich sein.

### Bezugszeichen

- 10: Motor
- 11: Anschlüsse
- 12: Welle
- 13, 15, 16: Permanentmagnete
- 14: Zwischenraum
- 17: Magnetfeldsensor
- 18: Kapsel
- 19: Flüssigkeit
- 20: magnetischer Körper, Dipol
- 21,22,26: Pfeile
- 23, 24, 25: Spulen
- 27: Gehäuse
- 28,29: Gehäuseschalen
- 30: Auswerte- und Steuereinheit
- 31: elektrische Verbindungen
- 32: Kontakte
- 33: Übertragungskapsel
- 34: Welle (mit Lagerung)
- 35: Förderschraube
- 36: Dipol
- 37: Induktionsspule
- 38: Wandung
- 39: Blähsack, Schalter
- 40: Antriebsabgriffe
- 41: Antriebsachsen
- 141: Halterung
- 251: Befestigungen
- 331,332: Teilräume
- 333,334: Steuerventile
- 341: Druckfläche
- M: Motor
- N: Nordpol
- S: Südpol

## Patentansprüche

1. Anordnung zur berührungslosen Bestimmung der Orientierung und Positionierung mindestens eines Gehäuses (27), und / oder zur berührungslosen Energieübertragung in das Gehäuse (27), mit
dem Gehäuse (27), in dem ein magnetischer Körper (20) in einer Kapsel (18) in wenigstens einer Dimension frei beweglich gelagert ist , wobei die Bewegung des magnetischen Körpers (20) damit von der Bewegung des mit der Kapsel (18) starr verbundenen Gehäuses (27) unabhängig und getrennt ist,
**gekennzeichnet durch** mindestens einen definiert beweglichen Permanentmagneten (13), wobei der ein magnetisches Moment aufweisende magnetische Körper (20) im Primärmagnetfeld des mindestens einen Permanentmagneten (13) angeordnet ist und ein vom magnetischen Körper (20) ausgehendes Sekundärmagnetfeld aufweist, wobei bei einer Bewegung des Primärmagnetfelds relativ zum magnetischen Körper (20) Veränderungen der Ausrichtung des Sekundärmagnetfelds bewirkt sind, und **dadurch** der magnetische Körper (20) definiert bewegt ist und und ferner **dadurch** gekennzeichnet,
dass die Kapsel (18) mit einer dreidimensional wirkenden Spulenanordnung (23, 24, 25) versehen ist, die an der Kapsel (18) befestigt ist.

2. Anordnung gemäß Anspruch 1, **dadurch gekennzeichnet, dass** zur Erfassung des Sekundärmagnetfeldes des magnetischen Körpers (20) mindestens ein Magnetfeldsensor (17) vorhanden ist.

3. Anordnung gemäß Anspruch 1 oder 2, **dadurch gekennzeichnet, dass** der magnetische Körper (20) ein kugelförmiger Dipol ist.

4. Anordnung gemäß einem der Ansprüche 1 bis 3, **dadurch gekennzeichnet, dass** der magnetische Körper (20) in einer Flüssigkeit (19) gelagert ist.

5. Anordnung gemäß einem der Ansprüche 1 bis 4, **dadurch gekennzeichnet, dass** der Permanentmagnet (13) rotationssymmetrisch gestaltet, radial magnetisiert und um seine Symmetrieachse drehbar gelagert ist.

6. Anordnung gemäß Anspruch 5, **dadurch gekennzeichnet, dass** der Magnetfeldsensor (17) dreidimensional wirksam ist.

7. Anordnung gemäß einem der Ansprüche 5 oder 6, **dadurch gekennzeichnet, dass** dem Magnetfeldsensor (17) eine Kompensationseinrichtung zugeordnet ist, die am Ort des Magnetfeldsensors (17) das Primärmagnetfeld kompensiert.

8. Anordnung gemäß einem der Ansprüche 5 bis 7, **dadurch gekennzeichnet, dass** die Kompensationseinrichtung aus einer Magnetanordnung besteht, die mit dem rotationssymmetrischen Permanentmagneten (13) fest verbunden und um die Symmetrieachse drehbar gelagert ist und die am Ort des Magnetfeldsensors (17) ein dem Primärmagnetfeld entgegengesetztes Feld gleicher Stärke erzeugt.

9. Anordnung gemäß Anspruch 7 oder 8, **dadurch gekennzeichnet, dass** die Kompensationseinrichtung mindestens eine elektrische Spule aufweist.

10. Anordnung gemäß Anspruch 1, **dadurch gekennzeichnet, dass** mehrere erfindungsgemäße Anordnungen nacheinander angeordnet sind und das jeweilige Sekundärmagnetfeld eines magnetischen Körpers (20) als Primärmagnetfeld zur Anregung weiterer magnetischer Körper (20) dient.

11. Anordnung gemäß Anspruch 6, **dadurch gekennzeichnet, dass** der Magnetfeldsensor (17) ein Magnetometer ist.

12. Anordnung gemäß einem der Ansprüche 1 oder 2, **dadurch gekennzeichnet, dass** die Spulenanordnung (23, 24, 25) und der magnetische Körper (20) einen Generator zur Energiegewinnung bilden, wobei die Energie einem Speicher oder mindestens einem Energieverbraucher zugeführt wird.

13. Anordnung gemäß Anspruch 12, **dadurch gekennzeichnet, dass** die einzelnen Spulen der Spulenanordnung (23, 24, 25) in Reihe geschaltet sind.

14. Anordnung gemäß einem der vorhergehenden Ansprüche, **dadurch gekennzeichnet, dass** in dem Gehäuse (27) einerseits die Kapsel (18) und andererseits eine Übertragungskapsel (33) fest angeordnet sind, wobei die Übertragungskapsel (33) eine Welle (34) aufweist, die mit der Drehachse des magnetischen Körpers (20) in der Kapsel (18) zumindest annähernd fluchtet.

15. Verwendung einer Anordnung gemäß einem der Ansprüche 1 bis 14, zur Messung bestimmter physikalischer und chemischer Eigenschaften eines Lagermediums des magnetischen Körpers (20).

16. Verwendung gemäß Anspruch 15, **dadurch gekennzeichnet, dass** aus einer nachlaufenden Phasenlage des Sekundärmagnetfeldes gegenüber dem Primärmagnetfeld die Viskosität der Flüssigkeit (19) bestimmt wird.

## Claims

1. Arrangement for non-contact determination of the orientation and positioning of at least one housing (27) and / or for non-contact energy transmission into the housing (27), with the housing (27) in which a magnetic body (20) is mounted in a capsule (18) in a freely movable manner in at least one dimension, wherein die movement of the magnetic body (20) is thereby independent of and separate from the movement of the housing (27) which is rigidly connected with the capsule (18), **characterized by** at least one permanent magnet (13) being movable in a defined manner, wherein the magnetic body (20) having a magnetic moment is arranged in the primary magnetic field of the at least one permanent magnet (13) and has a secondary magnetic field extending from the magnetic body (20), wherein changes in the alignment of the secondary magnetic field are caused by a movement of the primary magnetic field relative to the magnetic body (20), and thereby the magnetic body (20) is moved in a defined manner, and further **characterized in that** the capsule (18) is provided with a coil arrangement (23, 24, 25) having a three-dimensional effect, which is fastened to the capsule (18).

2. Arrangement according to claim 1, **characterized in that** at least one magnetic field sensor (17) is provided to record the secondary magnetic field of the magnetic body (20).

3. Arrangement according to claim 1 or 2, **characterized in that** the magnetic body (20) is a spherical dipole.

4. Arrangement according to one of claims 1 to 3, **characterized in that** the magnetic body (20) is mounted in a liquid (19).

5. Arrangement according to one of claims 1 to 4, **characterized in that** the permanent magnet (13) has a rotationally symmetrical design, is magnetized radially and is mounted so as to be rotatable about its symmetry axis.

6. Arrangement according to claim 5, **characterized in that** the magnetic field sensor (17) has a three-dimensional effect.

7. Arrangement according to one of claims 5 or 6, **characterized in that** a compensation device is associated with the magnetic field sensor (17), which compensates the primary magnetic field at the location of the magnetic field sensor (17).

8. Arrangement according to one of claims 5 to 7, **characterized in that** the compensation device comprises a magnet arrangement, which is fixedly connected to the rotationally symmetrical permanent magnet (13) and is mounted so as to be rotatable about the symmetry axis and which generates a reverse field of the same strength to the primary magnetic field at the location of the magnetic field sensor (17).

9. Arrangement according to claim 7 or 8, **characterized in that** the compensation device has at least one electric coil.

10. Arrangement according to claim 1, **characterized in that** several arrangements according to the invention are arranged one after the other and the secondary magnetic field of a magnetic body (20) serves as primary magnetic field for exciting further magnetic bodies (20).

11. Arrangement according to claim 6, **characterized in that** the magnetic field sensor (17) is a magnetometer.

12. Arrangement according to one of claims 1 or 2, **characterized in that** the coil arrangement (23, 24, 25) and the magnetic body (20) form a generator for energy generation, wherein the energy is supplied to a reservoir or at least to an energy consumer.

13. Arrangement according to claim 12, **characterized in that** the single coils of the coil arrangement (23, 24, 25) are connected in series.

14. Arrangement according to one of the preceding claims, **characterized in that** the capsule (18), on the one hand, and a transmission capsule (33), on the other hand, are fixedly arranged in the housing (27), wherein the transmission capsule (33) has a shaft (34), which is at least approximately aligned with the rotational axis of the magnetic body (20) in the capsule (18).

15. Use of an arrangement according to one of claims 1 to 14 for measuring certain physical and chemical properties of a storage medium of the magnetic body (20).

16. Use according to claim 15, **characterized in that** the viscosity of the liquid (19) is determined from a retarded phase position of the secondary magnetic field relative to the primary magnetic field.

## Revendications

1. Dispositif permettant la détermination sans contact de l'orientation et du positionnement d'au moins un boîtier (27) et / ou permettant la transmission d'énergie sans contact dans le boîtier (27), avec le boîtier (27) dans lequel est monté un corps magnétique (20) dans une capsule (18) de manière à pouvoir se déplacer librement dans au moins une dimension, le mouvement du corps magnétique (20) étant ainsi indépendant et séparé du mouvement du boîtier (27) qui est relié de manière rigide à la capsule (18),
**caractérisé par** au moins un aimant permanent (13) pouvant se déplacer de manière définie, le corps magnétique (20), qui a un moment magnétique, étant disposé dans le champ magnétique primaire de l'au moins un aimant permanent (13) et ayant un champ magnétique secondaire, qui s'étend à partir du corps magnétique (20), des changements de l'alignement du champ magnétique secondaire étant provoqués par un mouvement du champ magnétique primaire relative au corps magnétique (20), et ainsi le corps magnétique (20) étant déplacé de manière définie, et de plus **caractérisé en ce que** la capsule (18) est pourvue d'un ensemble de bobines (23, 24, 25) ayant un effet tridimensionnel, qui est fixé à la capsule (18).

2. Dispositif selon la revendication 1, **caractérisé en ce qu'**au moins un détecteur de champ magnétique (17) est prévu pour la détection du champ magnétique secondaire du corps magnétique (20).

3. Dispositif selon la revendication 1 ou 2, **caractérisé en ce que** le corps magnétique (20) est un dipôle sphérique.

4. Dispositif selon l'une des revendications 1 à 3, **caractérisé en ce que** le corps magnétique (20) est monté dans un liquide (19).

5. Dispositif selon l'une des revendications 1 à 4, **caractérisé en ce que** l'aimant permanent (13) est formé en symétrie de révolution, est magnétisé radialement et est monté de manière à pouvoir tourner autour de son axe de symétrie.

6. Dispositif selon la revendication 5, **caractérisé en ce que** le détecteur de champ magnétique (17) a un effet tridimensionnel.

7. Dispositif selon l'une des revendications 5 ou 6, **caractérisé en ce qu'**un dispositif de compensation est associé avec le détecteur de champ magnétique (17), qui compense le champ magnétique primaire à l'endroit du détecteur de champ magnétique (17).

8. Dispositif selon l'une des revendications 5 à 7, **caractérisé en ce que** le dispositif de compensation comprend un ensemble d'aimants, qui est relié de manière fixe à l'aimant permanent (13) à symétrie de révolution et qui est monté de manière à pouvoir tourner autour de l'axe de symétrie et qui génère un champ opposé de même force au champ magnétique primaire à l'endroit du détecteur de champ magnétique (17).

9. Dispositif selon la revendication 7 ou 8, **caractérisé en ce que** le dispositif de compensation a au moins une bobine électrique.

10. Dispositif selon la revendication 1, **caractérisé en ce que** plusieurs dispositifs selon l'invention sont disposés l'un après l'autre et le champ magnétique secondaire d'un corps magnétique (20) sert comme champ magnétique primaire pour exciter d'autres corps magnétiques (20).

11. Dispositif selon la revendication 6, **caractérisé en ce que** le détecteur de champ magnétique (17) est un magnétomètre.

12. Dispositif selon l'une des revendications 1 ou 2, **caractérisé en ce que** l'ensemble de bobines (23, 24, 25) et le corps magnétique (20) forment un générateur pour la génération d'énergie, l'énergie étant délivrée à un réservoir ou au moins à un consommateur d'énergie.

13. Dispositif selon la revendication 12, **caractérisé en ce que** les bobines individuelles de l'ensemble de bobines (23, 24, 25) sont connectées en série.

14. Dispositif selon l'une des revendications précédentes, **caractérisé en ce que** la capsule (18), d'un côté, et une capsule de transmission (33), d'un autre côté, sont disposées de manière fixe dans le boîtier (27), la capsule de transmission (33) ayant un arbre (34), qui est aligné au moins approximativement avec l'axe de rotation du corps magnétique (20) dans la capsule (18).

15. Usage d'un dispositif selon l'une des revendications 1 à 14, pour mesurer certaines propriétés physiques et chimiques d'un médium de stockage du corps magnétique (20).

16. Usage selon la revendication 15, **caractérisé en ce que** la viscosité du liquide (19) est déterminée d'une position de phase retardée du champ magnétique secondaire relatif au champ magnétique primaire.
